# NEUE EUROPÄISCHE PATENTSCHRIFT

(11) **EP 2 214 658 B2**
(45) Veröffentlichungstag und Bekanntmachung des Hinweises auf die Entscheidung über den Einspruch: **04.08.2021**
(45) Hinweis auf die Patenterteilung: 22.06.2011
(21) Anmeldenummer: 08843988.0
(22) Anmeldetag: 29.10.2008
(51) Int. Cl.: A61K 31/205, A61K 31/505, A61K 31/66, A61P 11/00, A61P 11/02, A61P 29/00, A61P 43/00

(54) **OSMOLYTHALTIGE ZUBEREITUNG ZUR ANWENDUNG BEI TROCKENEN SCHLEIMHÄUTEN**
OSMOLYTE-CONTAINING PREPARATION FOR USE IN CASE OF DRY MUCOUS MEMBRANES
PRÉPARATION CONTENANT DES OSMOLYTES DESTINÉE À ÊTRE EMPLOYÉE EN CAS DE MUQUEUSES SÈCHES

(30) Priorität: 31.10.2007 DE 102007052380
(43) Veröffentlichungstag der Anmeldung: 11.08.2010
(73) Patentinhaber: bitop AG, 58453 Witten (DE)
(72) Erfinder: SCHMITTMANN, Hans Bernd, 42551 Velbert (DE)
(74) Vertreter: Thiel, Christian
(86) Internationale Anmeldenummer: PCT/EP2008/009127
(87) Internationale Veröffentlichungsnummer: WO 2009/056292

(56) Entgegenhaltungen:
- WO-A-2005/002556
- WO-A-2005/002581
- WO-A-2007/146869
- WO-A-2008/031028
- WO-A1-02/24116
- WO-A1-2005/018601
- WO-A1-2005/094771
- WO-A2-02/19978
- DE-A1- 10 040 933
- DE-U1-202006 011 920
- US-A- 4 826 683
- Fachinformation 'Otriven' gegenSchnupfen 0,1% Novartis. Stand August 2007.
- J. Johnsen et al: Arch. Otolaryngol. Head Neck Surg., vol. 127, 2001, pages 1353-1356,
- C. Morgenstern: Der Arzneimittelbrief, vol. 25, no. 10, 1991, pages 73-77,
- X.Y. Su et al: International Journal of Pharmaceutics, vol. 123, 1995, pages 47-51,
- A.R. Talbot et al: The Laryngoscope, vol. 107, 1997, pages 500-503,

## Beschreibung

Die Nase erfüllt neben der Geruchswahrnehmung wichtige Aufgaben: Sie reinigt die Atemluft von kleinen Partikeln, erwärmt sie auf Körpertemperatur und befeuchtet sie. Auf diese Weise werden krankheitsauslösende Faktoren eliminiert und der Gasaustausch in der Lunge optimal vorbereitet. Dies kann aber nur funktionieren, wenn die Nasenschleimhaut in der Lage ist, ausreichend Feuchtigkeit an die Atemluft abzugeben. Ist die Luft jedoch besonders trocken (bei einer Luftfeuchtigkeit unter 5 g Wasser pro Kubikmeter Luft), wie im Winter oder in klimatisierten Räumen, dann ist die Kapazität der Nasenschleimhaut bald überlastet. Es kommt zu den Symptomen einer Rhinitis sicca (trockenen Nase) mit Juckreiz, Brennen, Ekzem- und Borkenbildung. Manchmal tritt auch Nasenbluten auf, oft ist der Nasengang auch verstopft, ohne dass ein Schnupfen vorliegt. Allein diese Symptome können schon sehr unangenehm sein. Doch die Folgen einer trockenen Nase resultieren vor allem aus ihrem Funktionsverlust: Die zu trockene und zu kalte, ungefilterte Luft trägt über kurz oder lang Krankheitserreger in den nunmehr ungeschützten Respirationstrakt.

Das "Syndrom der trockenen Nase", wovon eine Manifestation Rhinitis sicca und eine andere atrophische Rhinitis ist, ist ein ernstes medizinisches Problem. Es kann ebenfalls eine Nebenwirkung einer bestimmten medikamentösen Behandlung der Nase und längerem/wiederholtem Aufenthalt in klimatisierten Räumen sein. Ein großes Kontingent von Patienten mit trockener Nasenschleimhaut wird zudem durch starke Raucher gestellt (Zigaretten-Abusus).

Meist ist eine wässrige, isotonische Kochsalzlösung das Mittel der ersten Wahl, um eine trockene Nase zu behandeln. Doch die Behandlung in Sprayform wirkt nicht immer zufriedenstellend und muss recht häufig wiederholt werden. Verglichen mit anderen Nasalia bieten höherviskose Präparate charakteristische Vorteile: Gegenüber wasserhaltigen Nasentropfen oder -sprays verbleiben sie länger auf der Nasenschleimhaut. Somit ist der pflegende Effekt intensiver. Ein Nachteil der Verabreichung wässriger viskoser Präparate besteht aber darin, dass nach der Verdampfung des Wassers aus dem viskositätsergebenden Mittel eine unangenehme Kruste gebildet wird. Weiterhin ist eine sehr ernste Nebenwirkung oder ein Problem der subjektive Eindruck der "trockenen Nase", für die weiterhin kaum signifikante zufriedenstellende Behandlungen vorhanden sind. In der Patentanmeldung DE 43 04 893 werden Polyole (z.B. Glycerin, Glykol 300-1000, Polypropylenglykol 300-1000) in einem inerten Polymer, bevorzugt mit einem nicht-Newtonschen rheologischen Profil vorgeschlagen.

Hierbei wurde gefunden, dass man eine hohe Viskosität benötigt, um eine zufriedenstellende klinische Wirkung zu erhalten. Allerdings gelangen viskose Zubereitungen nur schwer deutlich weiter als nur bis zum Nasenvorhof, so dass höhere Areale der Nasenschleimhaut nur unzureichend behandelt werden.

Die Anwendung von Mineralölen in der Nase wird heute als bedenklich erachtet, da sie zu Granulomen (Knoten) der Nasenschleimhaut führen können. Die Knötchen entstehen bei dem vergeblichen Versuch der Nasenschleimhaut, das inerte Paraffin durch resorptive Prozesse zu entfernen. Auch kann es zum Einatmen des Paraffins mit intrapulmonaler Granulombildung kommen.

Eine wiederholte Anwendung von vasokonstriktorischen bzw. schleimhautabschwellenden Zusätzen (Sympathomimetika) führt andererseits häufig zu einer Austrocknung der Nasenschleimhäute, verbunden mit entzündlichen Irritationen. Diese Nebenwirkungen können zu einer erhöhten Infektionsgefahr führen, da die Schleimhäute im ausgetrockneten und entzündeten Zustand nicht mehr ihre Schutz- und Filterfunktionen in vollem Umfang aufrechterhalten können, so dass Krankheitserreger ungehindert in die Atemwege gelangen können. In den Schriften DE 195 41 919, DE 195 49 421 bzw. DE 103 56 248 werden daher Zusätze von Pantothenol oder Pantothensäure bzw. sauren Glykosaminoglykanen beschrieben. Dadurch werden die zuvor geschilderten Nachteile des Standes der Technik aber nur abgeschwächt. Die offenbarten Zusammensetzungen enthalten keine entzündungshemmenden Zusätze. Die verminderten entzündlichen Irritationen werden daher auf die bessere Befeuchtung der Nasenschleimhäute zurückgeführt. Um den allgemein bekannten Nebenwirkungen von Sympathomimetika besser entgegenwirken zu können, wird in der Gebrauchsmusterschrift DE 20 2006 005 924 die Verwendung von Myrrhe empfohlen. Im Rahmen der offenbarten Zusammensetzung soll die Myrrhe eine entzündungshemmende, antiphlogistische Wirkung entfalten. Ein Zusatz von Zinkverbindungen hilft den betroffenen Zellen, freie Radikale zu neutralisieren und unterstützt damit die Wirkungen der Myrrhe. Demnach wirkt hier allein die Kochsalzlösung einer Austrocknung der Schleimhäute entgegen.

Der vorliegenden Erfindung liegt daher die Aufgabe zugrunde, eine Zubereitung bereitzustellen, welche sich zur Prävention, Therapie und/oder Pflege von trockenen Nasenschleimhäuten eignet und insbesondere die zuvor geschilderten Nachteile des Standes der Technik überwindet.

Eine weitere Aufgabe der vorliegenden Erfindung liegt darin, eine Zubereitung bereitzustellen, welche jedoch ohne Sympathomimetika mit vasokonstriktorischen bzw. schleimhautabschwellenden Eigenschaften auskommt. Alternativ sollte die erfindungsgemäße Zubereitung das Austrocknen und die entzündlichen Irritationen der Nasenschleimhäute als typische Nebenwirkungen von Sympathomimetika mindern können.

Eine wünschenswerte Zubereitung muss demnach folgenden Forderungen genügen:
a) Höhere Salzkonzentrationen befeuchten nicht mehr die Schleimhäute, sondern entziehen ihnen sogar das Wasser. Vorteilhafter sind daher Verbindungen, welche auch in höheren Konzentrationen das Wasser nicht entziehen und zudem eine größere physiologische Verträglichkeit aufweisen.
b) Die Befeuchtung durch eine optionale Kochsalz-/Meersalzlösung bzw. meerwasserhaltige Lösung muss unterstützt und die oben beschriebene Nebenwirkung gemindert werden.
c) Die entzündungshemmenden Zusätze müssen besser auf eine ggf. salzhaltige Zubereitung abgestimmt sein.
d) Die Zusätze sollten in der Lage sein, weitere pharmakologische Wirkstoffe ggf. zu stabilisieren und/oder deren Nebenwirkungen zu mindern.

Der Erfinder hat nun überraschenderweise gefunden, dass diese Probleme dadurch gelöst werden können, dass man eine Zubereitung bzw. Formulierung auf Basis von Osmolyten oder Derivaten von Osmolyten nach Anspruch 1 oder 2 bereitstellt, der optional Natriumchlorid und/oder Moisturizer zugesetzt werden können. Als Moisturizer sind Scleroglucane (z.B. Tinocare) besonders vorteilhaft. Unter Derivaten werden die entsprechenden Salze oder Ester verstanden.

Die Zubereitung kann ebenfalls als Vehikel für eine Arzneimittelabgabe dienen.

Der Begriff "Zubereitung" bzw. "Formulierung" oder dergleichen, wie er im Rahmen der vorliegenden Erfindung verwendet wird, ist sehr weitgehend zu verstehen und umfasst nicht nur pharmazeutische Präparate bzw. Pharmazeutika als solche, sondern auch sogenannte Medizinprodukte oder dergleichen sowie Kosmetika.

Osmolyte bzw. kompatible Solute sind natürliche Wirkstoffe, die einen nebenwirkungsfreien Schutz der menschlichen Haut vor schädigenden Umwelteinflüssen ermöglichen (z.B. M. F. Roberts, "Organic compatible solutes of halotolerant and halophilic microorganisms", Saline Systems 2005, 1: 5, http://www.salinesystems.org/content/1/1/5). Die Osmolyte Ectoin und Hydroxyectoin schützen z.B. Zellstrukturen der menschlichen Haut und deren Erbmaterial vor Schädigungen infolge der Einwirkung von UV-Strahlung und anderen Formen von Umweltstress. Durch den Zellschutz der Ectoine bleibt die Immunantwort der Hautzellen und somit der Selbstschutzmechanismus der Haut vor nachhaltigen Schäden länger erhalten. Ectoine verzögern durch ihre Schutzfunktion Entzündungsreaktionen der Haut. Diese Eigenschaften der Ectoine sind durch zahlreiche Anwendungsstudien belegt und Ectoine sind in verschiedenen konventionellen kosmetischen Produkten bereits am Markt.

Ectoine zur Herstellung von Arzneimitteln werden in EP 0 887 418 erwähnt, ohne die Arzneimittel allerdings zu spezifizieren. Ectoinhaltige pharmazeutische Zubereitungen mit mindestens einer proteinhaltigen Subtanz (WO 00/76528) bzw. einem pharmazeutisch zulässigen Carrier (EP 0 553 884) sind ebenfalls bekannt.

Ectoine werden als natürlicher Zellschutzstoff aus extremophilen Mikroorganismen gewonnen. Extremophile Mikroorganismen zählen zu den ältesten Lebensformen auf der Erde und sind an drastische Umweltbedingungen wie extreme Temperaturen (teilweise über 100 °C) oder hohe Salzgehalte (200-300 g/l) optimal angepasst. Die natürlichen Lebensräume sind z.B. Salinen, heiße Quellen oder untermeerische Vulkane. Extremolyte sind essentiell für den Schutz verschiedener extremophiler Mikroorganismen gegen Stressfaktoren wie Kälte, Hitze, Salz, UV-Strahlung oder Radikale. Zu der erfindungsgemäßen Gruppe der Osmolyte gehören 1,4,5,6-Tetrahydro-2-methyl-pyrimidin-4-carbonsäure (Ectoin), 4,5,6,7-Tetrahydro-2-methyl-1H-[1,3]-diazepin-4-S-carbonsäure (Homoectoin), S,S-β-Hydroxy-1,4,5,6-Tetrahydro-2-methyl-pyrimidin-4-carbonsäure (Hydroxyectoin), Di-myo-Inositolphosphat (DIP), cyclisches 2,3-Diphosphoglycerat (cDPG), 1,1-Di-Glycerinphosphat (DGP), β-Mannosylglycerat (Firoin), β-Mannosylglyceramid (Firoin-A), Di-Mannosyl-di-inositolphosphat (DMIP) und Glucosylglycerin. Die aufgeführten Glycerinderivate haben gegenüber den in der Patentanmeldung DE 43 04 893 genannten Polyolen den Vorteil, dass sie eine weitaus größere physiologische Verträglichkeit aufweisen.

Die Osmolyte liegen typischerweise in einer Konzentration von 0,001 bis 50 Gew.-%, bevorzugt 0,05 bis 20 Gew.-%, insbesondere 0,1 bis 10 Gew.-% bezogen auf das Gesamtgewicht der Zusammensetzung vor.

Wie bereits erwähnt kann die Zusammensetzung auch Natriumchlorid in Form von Kochsalz, Meersalz oder Meerwasser enthalten. Bezogen auf einen Liter der Zusammensetzung beträgt der Gehalt beispielsweise 0,5 bis 20 g, insbesondere 1 bis 10 g, bevorzugt 2 bis 8 g, besonders bevorzugt 5 bis 7 g. Von Meerwasser ausgehend kann der Salzgehalt optional mit Kochsalz/Meersalz nachjustiert werden.

Die erfindungsgemäße Zubereitung kann eine flüssige oder dickflüssige bis halbfeste Konsistenz aufweisen. Beispielsweise kann die erfindungsgemäße Zusammensetzung als Salbe, Creme oder Gel zum Einbringen in die Nase oder bevorzugt als Lösung oder Dispersion zum Tropfen bzw. Sprühen in die Nase oder als Spüllösung vorliegen.

Als Träger für flüssige Darreichungsformen eignen sich insbesondere wässrige Systeme mit oder ohne Puffer. Als Trägerstoffe für dickflüssige oder halbfeste Zubereitungen, wie zum Beispiel Salben, Cremes oder Gele, eignen sich zum Beispiel Paraffinkohlenwasserstoffe, Vaseline, Wollwachsprodukte und andere pharmazeutisch verwendbare, viskositätserhöhende Grundstoffe, bei hydrophilen Gelen zum Beispiel Wasser, Glycerin oder Sorbit, die mit geeigneten Quellstoffen, wie z.B. Polyacrylsäure, Cellulosederivaten, Stärke oder Traganth, geliert werden. Insbesondere bei salzhaltigen Zusammensetzungen wird die Verdickung so gewählt, dass ein Durchlaufen in den Rachenraum weitgehend vermieden werden kann.

Die erfindungsgemäße Zubereitung kann neben den Wirk- und Trägersubstanzen und gegebenenfalls vorhandenen Emulgatoren noch andere unbedenkliche und in Bezug auf die Wirkstoffe kompatible pharmazeutische Hilfsstoffe und/oder Zusatzstoffe enthalten, so z.B. Füll, Streck-, Binde-, Netz-, Stabilisierungs-, Färbe-, Puffer-, Riech- und/oder Konservierungsstoffe.

In diesem Zusammenhang sind besonders die Zusätze von Tee bzw. Tee-Extrakten sowie Aloe vera zu nennen. Saponine besitzen als natürliche Netzmittel vielerlei Einsatzmöglichkeiten. Sie werden häufig wegen ihrer oberflächenaktiven Eigenschaften in der Kosmetik und in Lebensmitteln verwendet. Physiologisch ist ihre permeabilitätserhöhende und damit resorptionserhöhende Wirkung auf Membranen bekannt. Weiterhin können mikrobiologisch aktive chemische Verbindungen, wie z.B. Konservierungsstoffe, Antiseptika oder Manuca-Öl zur Verbesserung der mikrobiellen Stabilität, in üblichen Konzentrationen in der erfindungsgemäßen Zusammensetzung enthalten sein. Darüber hinaus kann die erfindungsgemäße Zusammensetzung auch noch eine oder mehrere pharmakologisch wirksame Substanzen enthalten. Als Konservierungsmittel können z. B. Sorbate, Benzoate oder Manuca-Öl eingesetzt werden. Die Konzentration liegt typischerweise in einem Bereich zwischen 0,02 und 5 Gew.-% bezogen auf das Gesamtgewicht der Zusammensetzung.

Die Zubereitungen können darüber hinaus zur Einstellung eines bestimmten pH-Wertes ein pH-Puffersystem aufweisen. Insbesondere kann es sich um ein Puffersystem auf Basis von Zitrat/Zitronensäure oder auf Phosphat-/Hydrogenphosphatbasis handeln.

Die Zusammensetzung kann auch als Vehikel für eine Arzneimittelabgabe dienen. Dabei können in der Zusammensetzung zusätzlich enthaltene Wirkstoffe stabilisiert und/oder deren Nebenwirkungen gemindert werden. Zusätzlich können durch die gemeinsame Verabreichung der Osmolyte gemäß der Erfindung und anderer Wirkstoffe synergistische Effekte bei der positiven Wirkung erzielt werden. So kann z. B. die abschwellende Wirkung von Oxymetazolin, Xylometazolin oder Tramazolin mit der Wirkung der Osmolyte kombiniert werden. Insbesondere kann die Wirkung der Osmolyte mit der antientzündlichen Wirkung von anderen Stoffen, wie z. B. Dexpanthenol oder Panthenol kombiniert werden. Ebenfalls möglich ist eine Kombination mit Antihistaminika wie Azelastin oder Cromoglicinsäure. Eine weitere Kombinationsmöglichkeit besteht mit viskositätserhöhenden Stoffen wie Hydroxypropylmethylcellulose, Hyetellose, Hypromellose oder Hyaluronsäure oder mit befeuchtenden Stoffen wie Sesamöl.

Neben der Zusammensetzung selbst betrifft die Erfindung auch die Verwendung von Osmolyten nach Anspruch 14 zur Herstellung eines Mittels zur kurativen topischen Behandlung bzw. nach Anspruch 15 zur prophylaktischen und/oder kurativen topischen Behandlung von trockenen Schleimhäuten, insbesondere Nasenschleimhäuten. Auf diese Weise kann ein Sekretstau ebenso wie das Auftreten von Austrocknungen und entzündlichen Irritationen der Schleimhäute verhindert werden. Des Weiteren dient die Behandlung der trockenen Schleimhäute der Reduktion von Ödembildung und der Verbesserung der nasalen Ventilation, insbesondere zur Belüftung der Nebenhöhlen und der Tuben.

Erfindungsgemäß ist auch das Vorsehen einer Inhalationsvorrichtung in Form eines gefüllten Inhalators für flüssige Zusammensetzungen gemäß der Erfindung möglich.

Die Herstellung der Zusammensetzung erfolgt in an sich bekannter Weise. Dies geschieht beispielsweise durch Mischen bzw. Lösen der Wirkstoffe in pharmakologisch wirksamen Konzentrationen, der Hilfs- und/oder Zusatzstoffe sowie der gegebenenfalls weiteren pharmakologisch wirksamen Substanzen in dem vorgesehenen Trägermedium.

Die folgenden Ausführungsbeispiele dienen lediglich der Veranschaulichung der vorliegenden Erfindung, ohne sie jedoch hierauf zu beschränken.

### Beispiel 1 [Ectoin, isotonisch in Wasser]:

In einem geeigneten Rührwerksbehälter werden ca. 45 % des vorgesehenen Endvolumens gereinigtes Wasser vorgelegt. Dann wird 3,87 % (w/w) Ectoin zugegeben und unter Rühren aufgelöst. Die entstandene Lösung wird mit gereinigtem Wasser auf ca. 98% des Endvolumens aufgefüllt und der pH-Wert mit 1 N Natronlauge/Milchsäure (Pural 80) auf pH 5,5-6,0 eingestellt. Die Lösung wird mit gereinigtem Wasser auf das vorgesehene Endvolumen aufgefüllt, durch einen geeigneten Filter filtriert und in Flaschen abgefüllt, welche anschließend mit einer geeigneten Nasenspraypumpe versehen werden.

### Beispiel 2 [Ectoin mit Salz, isotonisch in Wasser]:

In einem geeigneten Rührwerksbehälter werden ca. 45 % des vorgesehenen Endvolumens gereinigtes Wasser vorgelegt. Dann werden 0,5 % (w/w) Ectoin sowie 0,78 % (w/w) Kochsalz oder Meersalz zugegeben und unter Rühren aufgelöst. Die entstandene Lösung wird mit gereinigtem Wasser auf ca. 98 % des Endvolumens aufgefüllt und der pH-Wert mit 1 N Natronlauge/Milchsäure (Pural 80) auf pH 5,5-6,0 eingestellt. Die Lösung wird mit gereinigtem Wasser auf das vorgesehene Endvolumen aufgefüllt, durch einen geeigneten Filter filtriert und in Flaschen abgefüllt, welche anschließend mit einer geeigneten Nasenspraypumpe versehen werden.

### Beispiel 3:

In einem geeigneten Rührwerksbehälter werden ca. 45 % des vorgesehenen Endvolumens gereinigtes Wasser vorgelegt. Dann werden 0,5 % Ectoin, 0,78 % Kochsalz oder Meersalz sowie 4,9 % Tinocare SG-L (generischer Name Sclerotium gum) zugegeben und unter Rühren aufgelöst. Die entstandene Lösung wird mit gereinigtem Wasser auf ca. 98 % des Endvolumens aufgefüllt und der pH-Wert mit 1 N Natronlauge/Milchsäure (Pural 80) auf pH 5,5-6,0 eingestellt. Die Lösung wird mit gereinigtem Wasser auf das vorgesehene Endvolumen aufgefüllt, durch einen geeigneten Filter filtriert und in Flaschen abgefüllt, welche anschließend mit einer geeigneten Nasenspraypumpe versehen werden.

### Beispiel 4:

In einem geeigneten Rührwerksbehälter werden ca. 45 % des vorgesehenen Endvolumens gereinigtes Wasser vorgelegt. Dann werden 0,5 % Ectoin, 0,78 % Kochsalz, 0,1 % Saponin Q (DAB 9) sowie 4,8 % Tinocare SG-L zugegeben und unter Rühren aufgelöst. Die entstandene Lösung wird mit gereinigtem Wasser auf ca. 98 % des Endvolumens aufgefüllt und der pH-Wert mit 1 N Natronlauge/Milchsäure (Pural 80) auf pH 5,5-6,0 eingestellt. Die Lösung wird mit gereinigtem Wasser auf das vorgesehene Endvolumen aufgefüllt, durch einen geeigneten Filter filtriert und in Flaschen abgefüllt, welche anschließend mit einer geeigneten Nasenspraypumpe versehen werden.

### Beispiel 5 [Ectoin mit Salz in Tee]:

In einem temperierbaren Rührwerksbehälter werden ca. 45 % des vorgesehenen Endvolumens Tee (enthaltend 1,50 % Kamillentee oder Grünen Tee) vorgelegt. Den pH-Wert mit 1 N Natronlauge/Milchsäure (Pural 80) auf pH 5,5-6,0 einstellen. Dann werden 0,5% Ectoin, 2,00 % Kochsalz/Meersalz, 4,18 % Tinocare SG-L, 4,00 % Active-Aloe, 1,00 % Natriumasorbylphosphat, 0,20 % Kaliumsorbat, 0,10 % Saponin Q (DAB 9), 0,02 % Na-Hyaluronat sowie 0,50 % Glucosamininoglycan zugegeben und unter Rühren bei 45-50°C aufgelöst. Die entstandene Lösung wird mit 0,50 % Guar-Kernmehl versetzt und mit einem Dispergiergerät kurz vermengt, um die Klumpenbildung aufzuheben. Mit dem oben verwendeten Tee wird auf ca. 98 % des Endvolumens aufgefüllt und der pH-Wert mit 1 N Natronlauge/Milchsäure (Pural 80) auf pH 5,5-6,0 eingestellt. Nach ca. 24 Stunden Standzeit hat sich die anfängliche Trübung weitgehend geklärt. Die Lösung wird mit dem oben verwendeten Tee auf das vorgesehene Endvolumen aufgefüllt, durch einen geeigneten Filter filtriert und in geeignete Pipettenflaschen abgefüllt.

### Beispiel 6 [Ectoin ohne Salz in Tee]:

In einem temperierbaren Rührwerksbehälter werden ca. 45 % des vorgesehenen Endvolumens Tee (enthaltend 1,50 % Kamillentee oder Grünen Tee) vorgelegt. Der pH-Wert wird mit 1 N Natronlauge/Milchsäure (Pural 80) auf pH 5,5-6,0 eingestellt. Dann werden 0,5 % Ectoin, 5,00 % Tinocare SG-L, 5,00 % Active-Aloe, 0,50 % Natriumasorbylphosphat, 0,20 % Kaliumsorbat, 0,20 % Saponin Q (DAB 9), 0,02% Na-Hyaluronat sowie 0,50 % Glucosamininoglycane zugegeben und unter Rühren bei 45-50°C aufgelöst. Die entstandene Lösung wird mit 0,48 % Guar-Kernmehl versetzt und mit einem Dispergiergerät kurz vermengt, um die Klumpenbildung aufzuheben. Mit dem oben verwendeten Tee wird auf ca. 98 % des Endvolumens aufgefüllt und der pH-Wert mit 1 N Natronlauge/Milchsäure (Pural 80) auf pH 5,5-6,0 einstellen. Nach ca. 24 Stunden Standzeit hat sich die anfängliche Trübung weitgehend geklärt. Die Lösung wird mit dem oben verwendeten Tee auf das vorgesehene Endvolumen aufgefüllt, durch einen geeigneten Filter filtriert und in geeignete Pipettenflaschen abgefüllt.

### Anwendungsstudien

Die Wirksamkeit einer erfindungsgemäßen Ectoin-Lösung wurde anhand von 50 ambulanten Patienten mit der Diagnose Rhinitis sicca anterior untersucht. Die Behandlungsdauer betrug zwei Wochen. Den Patienten wurde empfohlen das Ectoin-Nasenspray mindestens 5 x täglich zu applizieren.

Als Hauptzielparameter wurde die subjektive Befindlichkeitsskala der Nasenatmungsbehinderung nach den Scores 0 bis 12 (0 - keine, 3 - geringe, 6 - mittlere, 9 - starke und 12 - sehr starke) entsprechend den Angaben der Patienten dokumentiert sowie das Ausmaß der Borkenbildung nach den Scores 0 bis 12 (0 - keine, 3 - geringe, 6 - mittlere, 9 - starke und 12 - sehr starke) befundet. Als Nebenzielparameter erfolgte ergänzend eine Bewertung der endonasalen Blutauflagerungen, der Ausprägung einer Begleitpharyngitis, der Geruchsbelästigung, der Rhinorrhoe, der Sekretviskosität und der Nasenmuschelhyperplasie. Auch zur Quantifierung der Nebenzielparameter diente die Scoreinteilung von 0 bis 12.

Nach einer oder zwei Wochen Therapiedauer wurde ebenfalls die Wirksamkeit, Verträglichkeit und Patientencompliance nach den Scores von 0 bis 12 (0 - sehr gute, 3 - gute, 6 - ausreichende, 9 - geringe und 12 - keine/schlechte) vom Untersucher protokolliert.

### Hauptzielparameter Nasenatmungsbehinderung:

Es wurden folgende Scores ermittelt:

| | |
|---|---|
| vor der Behandlung | 4,6 |
| nach einer Woche | 2,76 |
| nach zwei Wochen | 1,54 |

Die Veränderung der Nasenatmungsbehinderung war hoch signifikant (p < 0,001).

### Hauptzielparameter Borkenbildung / Trockenheitsgefühl im Naseninneren:

Es wurden folgende Scores ermittelt:

| | |
|---|---|
| vor der Behandlung | 6,2 |
| nach einer Woche | 2,16 |
| nach zwei Wochen | 1,52 |

Der Rückgang war hoch signifikant (p < 0,001).

### Nebenzielparameter Blutauflagerungen:

Es wurden folgende Scores ermittelt:

| | |
|---|---|
| vor der Behandlung | 2,14 |
| nach einer Woche | 0,36 |
| nach zwei Wochen | 0,36 |

Die Veränderung ist erneut als statistisch hoch signifikant einzustufen (p < 0,001).

### Nebenzielparameter Pharyngitis:

Es wurden folgende Scores ermittelt:

| | |
|---|---|
| vor der Behandlung | 1,08 |
| nach einer Woche | 0,34 |
| nach zwei Wochen | 0,16 |

Dieser Rückgang war ebenfalls statistisch hoch signifikant (p < 0,001).

### Nebenzielparameter Kakosmie:

Das Auftreten einer Kakosmie (n = 2) ist lediglich vor der Behandlung in der ärztlichen Befunddokumentation beschrieben worden. Dieses Symptom wurde nur von wenigen Patienten beschrieben, weshalb hier keine statistisch signifikanten Unterschiede abgeleitet werden konnten.

### Nebenzielparameter Rhinorrhoe:

Es wurden folgende Scores ermittelt:

| | |
|---|---|
| vor der Behandlung | 1,64 |
| nach einer Woche | 1,6 |
| nach zwei Wochen | 0,94 |

Dieses Ergebnis war jedoch statistisch nicht signifikant (p = 0,248).

### Nebenzielparameter Sekretviskosität:

Es wurden folgende Scores ermittelt:

| | |
|---|---|
| vor der Behandlung | 5,26 |
| nach einer Woche | 2,44 |
| nach zwei Wochen | 2,00 |

Der Rückgang der Symptome ist statistisch hoch signifikant (p < 0,001).

### Beurteilung der Wirksamkeit, Verträglichkeit und Compliance der Patienten:

Scoreinschätzung von ärztlicher Seite:

| | Wirksamkeit | Verträglichkeit | Compliance der Patienten |
|---|---|---|---|
| nach einer Woche | 3,86 | 2,16 | 2,30 |
| nach zwei Wochen | 3,50 | 2,08 | 2,12 |

Beurteilung der Wirksamkeit und Verträglichkeit durch die Patienten:

| | Wirksamkeit | Verträglichkeit |
|---|---|---|
| nach 3 Tagen | 4,58 | 2,10 |
| nach 6 Tagen | 4,14 | 1,92 |
| nach 9 Tagen | 3,46 | 1,60 |
| nach 12 Tagen | 3,12 | 1,40 |

Die Ergebnisse waren statistisch hoch signifikant.

Weitere Ausgestaltungen, Abwandlungen und Variationen sowie Vorteile der vorliegenden Erfindung sind für den Fachmann beim Lesen der Beschreibung ohne weiteres erkennbar und realisierbar, ohne dass er dabei den Rahmen der vorliegenden Erfindung verlässt.

## Patentansprüche

1. Zusammensetzung enthaltend als Wirkstoff mindestens einen Osmolyt zur kurativen topischen Behandlung von trockenen Schleimhäuten, wobei es sich bei dem Osmolyt um 1,4,5,6-Tetrahydro-2-methyl-pyrimidin-4-carbonsäure (Ectoin), S,S-ß-Hydroxy-1,4,5,6-Tetrahydro-2-methyl-pyrimidin-4-carbonsäure (Hydroxyectoin) und/oder ein Salz oder einen Ester dieser Verbindungen handelt.

2. Zusammensetzung enthaltend als Wirkstoff mindestens einen Osmolyt zur Behandlung von trockenen Schleimhäuten, wobei es sich bei dem Osmolyt um 4,5,6,7-Tetrahydro-2-methyl-1H-[1,3]-diazepin-4-S-carbonsäure (Homoectoin), Di-myo-Inositolphosphat (DIP), zyklisches 2,3-Diphosphoglycerat (cDPG), 1,1-Di-Glycerinphosphat (DGP), β-Mannosylglycerat (Firoin), β-Mannosylglyceramid (Firoin A), Di-Mannosyl-di-inositolphosphat (DMIP), Glucosylglycerin und/oder ein Salz oder einen Ester dieser Verbindungen handelt.

3. Zusammensetzung nach Anspruch 1 oder 2, **dadurch gekennzeichnet, dass** der Wirkstoff der Behandlung von trockenen Nasenschleimhäuten dient.

4. Zusammensetzung nach einem der Ansprüche 1 bis 3, **dadurch gekennzeichnet, dass** die Zusammensetzung Natriumchlorid enthält.

5. Zusammensetzung nach Anspruch 4, **dadurch gekennzeichnet, dass** die Zusammensetzung Kochsalz und/oder Meersalz in einer Menge von 0,5 bis 20 g, insbesondere 1 bis 10 g, bevorzugt 2 bis 8 g und besonders bevorzugt 5 bis 7 g, bezogen auf einen Liter der Zusammensetzung enthält.

6. Zusammensetzung nach einem der Ansprüche 1 bis 5, **dadurch gekennzeichnet, dass** die Zusammensetzung einen Moisturizer, bevorzugt ein Scleroglucan, insbesondere Tinocare, enthält.

7. Zusammensetzung nach einem der Ansprüche 1 bis 6, **dadurch gekennzeichnet, dass** die Osmolyte in Konzentrationen von 0,001 bis 50 Gew.-%, bevorzugt 0,05 bis 20 Gew.-%, insbesondere 0,1 bis 10 Gew.-%, bezogen auf das Gesamtgewicht der Zusammensetzung vorliegen.

8. Zusammensetzung nach einem der Ansprüche 1 bis 7, **dadurch gekennzeichnet, dass** die Zusammensetzung Sorbate, Benzoate und/oder Manuca-Öl in einer Konzentration von 0,02 bis 5 Gew.-% als Konservierungsmittel enthält.

9. Zusammensetzung nach einem der Ansprüche 1 bis 8, **dadurch gekennzeichnet, dass** die Zusammensetzung Aloe vera, Tee und/oder Teeextrakte enthält.

10. Zusammensetzung nach einem der Ansprüche 1 bis 9, **dadurch gekennzeichnet, dass** die Zusammensetzung Oxymetazolin, Xylometazolin, Tramazolin, Dexpanthenol, Panthenol, Sesamöl, Cromoglicinsäure, Azelastin, Hydroxypropylmethylcellulose, Hyetellose, Hypromellose, Hyaluronsäure, ein Salz oder einen Ester dieser Verbindungen oder eine Kombination der vorgenannten Stoffe enthält.

11. Zusammensetzung nach einem der Ansprüche 1 bis 10, **dadurch gekennzeichnet, dass** die Zusammensetzung eine wässerige Lösung ist.

12. Zusammensetzung nach einem der Ansprüche 1 bis 11, **dadurch gekennzeichnet, dass** es sich bei der Zusammensetzung um eine Lösung, Spülung, Suspension, Salbe, Creme, Lotion, Paste, ein Spray, Gelee, Aerosol, Nasenspray oder Nasentropfen handelt.

13. Zusammensetzung nach einem der Ansprüche 1 bis 12, **dadurch gekennzeichnet, dass** es sich um eine isotonische oder hypertonische Zusammensetzung handelt.

14. Verwendung mindestens eines Osmolyts zur Herstellung eines Mittels zur kurativen topischen Behandlung von trockenen Schleimhäuten, insbesondere Nasenschleimhäuten, wobei es sich bei dem Osmolyt um 1,4,5,6-Tetrahydro-2-methyl-pyrimidin-4-carbonsäure (Ectoin), S,S-ß-Hydroxy-1,4,5,6-Tetrahydro-2-methyl-pyrimidin-4-carbonsäure (Hydroxyectoin) und/oder ein Salz oder einen Ester dieser Verbindungen handelt.

15. Verwendung mindestens eines Osmolyts zur Herstellung eines Mittels zur prophylaktischen und/oder kurativen topischen Behandlung von trockenen Schleimhäuten, insbesondere Nasenschleimhäuten, wobei es sich bei dem Osmolyt um 4,5,6,7-Tetrahydro-2-methyl-1H-[1,3]-diazepin-4-S-carbonsäure (Homoectoin), Di-myo-Inositolphosphat (DIP), zyklisches 2,3-Diphosphoglycerat (cDPG), 1,1-Di-Glycerinphosphat (DGP), β-Mannosylglycerat (Firoin), β-Mannosylglyceramid (Firoin A), Di-Mannosyl-di-inositolphosphat (DMIP), Glucosylglycerin und/oder ein Salz oder einen Ester dieser Verbindungen handelt.

16. Verwendung nach Anspruch 14 oder 15, **dadurch gekennzeichnet, dass** das Mittel der Verhinderung eines Sekretstaus, der Verhinderung von Austrocknungen und/oder der Verhinderung entzündlicher Irritationen der Schleimhäute dient.

17. Verwendung nach einem der Ansprüche 14 bis 16, **dadurch gekennzeichnet, dass** das Mittel der Reduktion der Ödembildung und/oder der Verbesserung der nasalen Ventilation, insbesondere der Belüftung der Nebenhöhlen und der Tuben dient.

## Claims

1. Composition containing as active agent at least one osmolyte for the curative topical treatment of dry mucosa, wherein the osmolyte is 1,4,5,6-tetrahydro-2-methyl-pyrimidine-4-carboxylic acid (ectoine), S,S-β-hydroxy-1,4,5,6-tetrahydro-2-methyl-pyrimidine-4-carboxylic acid (hydroxyectoine), and/or a salt or ester of said compounds.

2. Composition, containing as an active agent at least one osmolyte for the treatment of dry mucosa, wherein the osmolyte is 4,5,6,7-tetrahydro-2-methyl-1H-[1,3]-diazepine-4-S-carboxylic acid (homoectoine), di-myo-inositol phosphate (DIP), cyclic 2,3-diphosphoglycerate (cDPG), 1,1-di-glycerol phosphate (DGP), β-mannosyigiycerate (firoin), β-mannosyigiyceramide (firoin-A), di-mannosyl-di-inositol phosphate (DMIP), glucosylglycerol and/or a salt or ester of said compounds.

3. Composition according to claim 1 or 2, **characterized in that** the active agent serves for the treatment of dry nasal mucosa.

4. Composition according to claim 1 to 3, **characterized in that** the composition contains sodium chloride.

5. Composition according to claim 4, **characterized in that** the composition contains common salt and/or sea salt in an amount of between 0.5 and 20 g, in particular 1 to 10 g, preferably 2 to 8 g and especially preferred 5 to 7 g, based on one liter of the composition.

6. Composition according to any one of claims 1 to 5, **characterized in that** the composition contains a moisturizer, preferably a scleroglucane, especially Tinocare.

7. Composition according to any one of claims 1 to 6, **characterized in that** the osmolytes have a concentration ranging between 0.001 and 50 % w/w, preferably 0.05 to 20 % w/w, in particular 0.1 to 10 % w/w based on the total weight of the composition.

8. Composition according to any one of claims 1 to 7, **characterized in that** the composition contains sorbates, benzoates and/or manuca oil of a concentration ranging between 0.02 and 5 % w/w as preservation agents.

9. Composition according to any one of claims 1 to 8, **characterized in that** the composition contains aloe vera, tea and/or tea extracts.

10. Composition according to any one of claims 1 to 9, **characterized in that** the preparation contains oxymetazoline, xylometazoline, tramazoline, dexpanthenol, panthenol, sesame oil, cromoglicic acid, azelastine, hydroxypropyl methylcellulose, hyetellose, hypromellose, hyaluronic acid, a salt or ester of these compounds or a combination of the aforementioned substances.

11. Composition according to any one of claims 1 to 10, **characterized in that** the composition is an aqueous solution.

12. Composition according to any one of claims 1 to 11, **characterized in that** the composition is provided in the form of a solution, irrigation, suspension, ointment, cream, lotion, paste, spray, jelly, aerosol, nasal spray or nose drops.

13. Composition according to any one of claims 1 to 12, **characterized in that** it is provided in the form of an isotonic or hypertonic composition.

14. Use of at least one osmolyte for the manufacture of an agent for the curative topical treatment of dry mucosa, in particular of nasal mucosa, wherein the osmolyte is 1,4,5,6-tetrahydro-2-methylpyrimidine-4-carboxylic acid (ectoine), S,S-β-hydroxy-1,4,5,6-tetrahydro-2-methyl-pyrimidine-4-carboxylic acid (hydroxyectoine) and/or a salt or ester of said compounds.

15. Use of at least one osmolyte for the manufacture of an agent for the prophylactic and/or curative topical treatment of dry mucosa, in particular of nasal mucosa, wherein the osmolyte is 4,5,6,7-tetrahydro-2-methyl-1H-[1,3]-diazepine-4-S-carboxylic acid (homoectoine), di-myo-inositol phosphate (DIP), cyclic 2,3-diphosphoglycerate (cDPG), 1,1-di-glycerol phosphate (DGP), β-mannosylglycerate (firoin), β-mannosylglyceramide (firoin-A), di-mannosyl-di-inositol phosphate (DMIP), glucosylglycerol and/or a salt or ester of said compounds.

16. Use according to claim 14 or 15, **characterized in that** the agent serves to prevent a secretion build-up, the occurrence of desiccation and/or inflammatory irritations of mucosa.

17. Use according to one of claims 14 to 16, **characterized in that** the agent serves to reduce the formation of oedemas and/or improve the nasal ventilation, especially ventilation of the paranasal sinuses and tubes.

## Revendications

1. Composition contenant comme principe actif au moins un osmolyte pour le traitement topique curatif de muqueuses sèches, l'osmolyte étant l'acide 1,4,5,6-tétrahydro-2-méthyl-pyrimidino-4-carboxylique (ectoïne), l'acide S,S-β-hydroxy-1,4,5,6-tétrahydro-2-méthyl-pyrimidino-4-carboxylique (hydroxyectoïne) et/ou un sel ou un ester de ces composés.

2. Composition contenant comme principe actif au moins un osmolyte pour le traitement de muqueuses sèches, l'osmolyte étant l'acide 4,5,6,7-tétrahydro-2-méthyl-1H-[1,3]-diazépino-4-S-carboxylique (homoectoïne), le di-myo-inositol phosphate (DIP), le 2,3-diphosphoglycérate cyclique (cDPG), le 1,1-di-glycérol phosphate (DGP), le β-mannosyl glycérate (firoïne), le β-mannosyl glycéramide (firoïne A), le di-mannosyl-di-inositol phosphate (DMIP), le glucosyl glycérol et/ou un sel ou un ester de ces composés.

3. Composition selon la revendication 1 ou 2, **caractérisée en ce que** le principe actif sert au traitement de muqueuses nasales sèches.

4. Composition selon l'une quelconque des revendications 1 à 3, **caractérisée en ce que** la composition contient du chlorure de sodium.

5. Composition selon la revendication 4, **caractérisée en ce que** la composition contient du sel de cuisine et/ou du sel de mer en une quantité de 0,5 à 20 g, en particulier de 1 à 10 g, de préférence de 2 à 8 g et de façon particulièrement préférée de 5 à 7 g, rapportés à un litre de la composition.

6. Composition selon l'une quelconque des revendications 1 à 5, **caractérisée en ce que** la composition contient un humidifiant, de préférence un scléroglucane, en particulier du Tinocare.

7. Composition selon l'une quelconque des revendications 1 à 6, **caractérisée en ce que** les osmolytes sont présents en des concentrations de 0,001 à 50 % en poids, de préférence de 0,05 à 20 % en poids, en particulier de 0,1 à 10 % en poids, rapportés au poids total de la composition.

8. Composition selon l'une quelconque des revendications 1 à 7, **caractérisée en ce que** la composition contient comme agents de conservation des sorbates, des benzoates et/ou de l'huile de Manuca en une concentration de 0,02 à 5 % en poids.

9. Composition selon l'une quelconque des revendications 1 à 8, **caractérisée en ce que** la composition contient de l'aloe vera, du thé et/ou des extraits de thé.

10. Composition selon l'une quelconque des revendications 1 à 9, **caractérisée en ce que** la composition contient de l'oxymétazoline, de la xylométazoline, de la tramazoline, du dexpanthénol, du panthénol, de l'huile de sésame, de l'acide chromoglycinique, de l'azélastine, de l'hydroxypropylméthylcellulose, de la hyétellose, de l'hypromellose, de l'acide hyaluronique, un sel ou un ester de ces composés ou une combinaison des substances susmentionnées.

11. Composition selon l'une quelconque des revendications 1 à 10, **caractérisée en ce que** la composition est une solution aqueuse.

12. Composition selon l'une quelconque des revendications 1 à 11, **caractérisée en ce qu'**il s'agit en matière de composition d'une solution, d'un rinçage, d'une suspension, d'une pommade, d'une crème, d'une lotion, d'une pâte, d'un spray, d'une gelée, d'un aérosol, d'un spray nasal ou de gouttes pour le nez.

13. Composition selon l'une quelconque des revendications 1 à 12, **caractérisée en ce qu'**il s'agit d'une composition isotonique ou hypertonique.

14. Utilisation d'au moins un osmolyte pour la préparation d'un produit pour le traitement curatif topique de muqueuses sèches, en particulier de muqueuses nasales sèches, l'osmolyte étant l'acide 1,4,5,6-tétrahydro-2-méthyl-pyrimidino-4-carboxylique (ectoïne), l'acide S,S-β-hydroxy-1,4,5,6-tétrahydro-2-méthyl-pyrimidino-4-carboxylique (hydroxyectoïne) et/ou un sel ou un ester de ces composés.

15. Utilisation d'au moins un osmolyte pour la préparation d'un produit pour le traitement prophylactique et/ou curatif topique de muqueuses sèches, en particulier de muqueuses nasales, l'osmolyte étant l'acide 4,5,6,7-tétrahydro-2-méthyl-1H-[1,3]-diazépino-4-S-carboxylique (homoectoïne), le di-myo-inositol phosphate (DIP), le 2,3-diphosphoglycérate cyclique (cDPG), le 1,1-di-glycérol phosphate (DGP), le β-mannosyl glycérate (firoïne), le β-mannosyl glycéramide (firoïne A), le di-mannosyl-di-inositol phosphate (DMIP), le glucosyl glycérol et/ou un sel ou un ester de ces composés.

16. Utilisation selon la revendication 14 ou 15, **caractérisée en ce que** le produit sert à empêcher une obstruction par sécrétions, le dessèchement et/ou des irritations inflammatoires des muqueuses.

17. Utilisation selon l'une quelconque des revendications 14 à 16, **caractérisée en ce que** le produit sert à la réduction de la formation d'oedèmes et/ou à l'amélioration de la ventilation nasale, en particulier de la ventilation des sinus et des trompes.
